# EUROPEAN PATENT APPLICATION

(11) **EP 4 268 835 A1**
(43) Date of publication of application: **01.11.2023**
(21) Application number: 21915478.8
(22) Date of filing: 03.11.2021
(51) Int. Cl.: A61K 35/74, A61K 9/00, A61P 11/00, A61P 1/16, A61P 35/00, A61P 13/12, A61P 25/28, A61P 29/00, A61P 37/08, A23L 33/135

(54) **COMPOSITION FOR PREVENTION OR TREATMENT OF NEUTROPHILIC PULMONARY DISEASE COMPRISING EXTRACELLULAR VESICLES DERIVED FROM MICROCOCCUS LUTEUS**

(30) Priority: 28.12.2020 KR 20200184337; 02.11.2021 KR 20210148789
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2021/015764
(87) International publication number: WO 2022/145680

(57) **Abstract**

The present invention relates to extracellular vesicles derived from Micrococcus luteus and use thereof, and particularly, to a composition and the like for ameliorating, preventing or treating neutrophilic pulmonary diseases, comprising *Micrococcus luteus-derived* vesicles as an active ingredient, the composition being capable of effectively treating a neutrophilic pulmonary disease mediated by a nucleotide-binding oligomerization domain, leucine rich repeat and pyrin domain containing 3 inflammasome (NLRP3 inflammasome).

## Description

### [Technical Field]

The present invention relates to extracellular vesicles derived from *Micrococcus luteus* and use thereof, and more particularly to a composition and the like for preventing or treating neutrophilic pulmonary disease, comprising *Micrococcus luteus-derived* vesicles as an active ingredient.

This application claims priority to and the benefit of Korean Patent Application Nos. 10-2020-0184337 and 10-2021-0148789 filed in the Korean Intellectual Property Office on December 28, 2020 and November 2, 2021, respectively, and all the contents disclosed in the specification and drawings of the applications are incorporated in this application.

### [Background Art]

As the 21st century begins, the importance of acute infectious diseases that were recognized as epidemics in the past has decreased, whereas chronic diseases whose main pathogenesis is inflammation caused by immune dysfunction occurring in the major organs of our body have changed the pattern of diseases to become a major disease that reduces the quality of life and determines a human's life expectancy.

Immunity is a defense mechanism of cells against biological, chemical, physical, and mental stress, and occurs through innate immunity and adaptive immunity. Recently, it has been known that pathogen-associated molecular patterns (PAMPs) derived from biological causative factors and damage-associated molecular patterns (DAMPs), which are danger signals generated by cell damage, are identified by pattern recognition receptors (PRRs) in relation to the pathogenesis of inflammatory diseases, and thus initiate inflammation. Among PRRs, a nucleotide-binding oligomerization domain-like receptor (NOD-like receptor; NLR) is a PRR that recognizes PAMPs and DAMPs in cells, and is a key material in inflammatory responses or cell death. A nucleotide-binding oligomerization domain, leucine rich repeat and pyrin domain containing 3 (NLRP3) inflammasome is a type of NOD-like receptor, recognizes PAMPs or DAMPs present in cells to form inflammasomes, and causes an inflammatory response through K+efflux and caspase-1 activation. Recently, attention has been focused on the fact that the NLRP3 inflammasome is an important signaling system in the pathogenesis of various intractable inflammatory diseases.

Meanwhile, it is known that the number of microorganisms that coexist in the human body reaches 100 trillion, which is about more than that of human cells, and the number of genes of microorganisms is 100-fold larger than that of humans. A microbiota or microbiome refers to a microbial community including bacteria, archaea and eukarya present in a given habitat.

Bacteria that coexist in our bodies and bacteria that exist in the surrounding environment secrete nanometer-sized vesicles to exchange information such as genes, low molecular compounds, and proteins with other cells. The mucosa forms a physical defense membrane through which particles having a size of 200 nanometers (nm) or more cannot pass, so that bacteria coexisting in the mucosa cannot pass through the mucosa, but bacteria-derived vesicles have a size of 200 nanometers or less, and thus relatively freely pass through epithelial cells of the mucosa to be absorbed in our bodies. As described above, although bacteria-derived vesicles are secreted from bacteria, they differ from bacteria in terms of their constituents, absorption rate in the body, and risk of side effects, and therefore, the use of bacteria-derived vesicles is completely different from that of living cells or has a significant effect.

Locally secreted bacteria-derived vesicles are absorbed through the epithelial cells of the mucosa to induce a local inflammatory response, and vesicles that have passed through the epithelial cells are systemically absorbed through the lymphatic vessels to be distributed to respective organs, and regulate immune and inflammatory responses in the organs to which the vesicles are distributed. For example, vesicles derived from pathogenic gram-negative bacteria, such as *Escherichia coli,* are pathogenic nanoparticles that cause local colitis, are absorbed into vascular endothelial cells when absorbed by blood vessels to promote a systemic inflammatory response and blood coagulation by inducing an inflammatory response, and are also absorbed into muscle cells where insulin acts to cause insulin resistance and diabetes mellitus. In contrast, vesicles derived from beneficial bacteria may regulate diseases by regulating immune and metabolic dysfunctions caused by pathogenic vesicles.

*Micrococcus luteus* is a gram-positive bacterium belonging to the genus Micrococcus, and a bacterium that is widely distributed in nature such as water, dust, and soil. The bacterium has catalase, and thus converts hydrogen peroxide, which is an active oxygen, into water and produces riboflavin when grown in toxic organic pollutants such as pyridine. Further, it has a lutein pigment which exhibits UV absorption and antioxidant effects. In addition, it is known that the bacterium is also isolated from dairy products and beer, grows in dry or high-salt environments, and does not form spores, but survives at a refrigeration temperature in a refrigerator for a long period of time.

In the dust we breathe, various biological causative factors such as viruses, bacteria-derived vesicles, and lipopolysaccharide (LPS), which induce inflammation in the lungs, are present. The repeated inhalation of these causative factors induces inflammation in the lungs because inflammatory mediators are secreted from pulmonary epithelial cells and macrophages by the PRR-mediated signaling system, and repeated stress causes neutrophilic pulmonary inflammatory diseases. Common compositions used to treat or prevent neutrophilic pulmonary inflammatory diseases broadly include steroidal and non-steroidal compositions, and recently, there has been increasing interest in TNF-α inhibitors, which are inflammatory cytokines known to be major mediators of inflammatory diseases.

However, there has been no report in which vesicles derived from *Micrococcus luteus* are applied to the treatment of neutrophilic pulmonary diseases to date.

### [Disclosure]

### [Technical Problem]

As a result of intensive studies to solve the aforementioned problems in the related art, the present inventors have confirmed that when *Micrococcus luteus-*derived vesicles are orally or intranasally administered, the vesicles pass through the mucosa and are distributed in the lungs, and confirmed that the secretion of inflammatory mediators by pathogenic nanoparticles was remarkably suppressed during the treatment with the vesicles. Furthermore, the present inventors confirmed that *Micrococcus luteus-derived* vesicles not only efficiently suppress immune dysfunction caused by biological causative factors, but also regulate immune dysfunction by suppressing the expression of NLRP3 protein, which is a pattern recognition receptor (PRR) associated with the pathogenesis of various diseases and increase cellular homeostasis by increasing endothelial NO synthase (eNOS) signaling. Further, the present inventors confirmed that when a mouse model of neutrophilic pulmonary disease caused by bacterial-derived vesicles and LPS-contaminated allergens was treated with *Micrococcus luteus-derived* vesicles, neutrophilic pulmonary inflammation and airway hyperresponsiveness were significantly suppressed, thereby completing the present invention based on this.

Thus, an object of the present invention is to provide a pharmaceutical composition for preventing or treating a neutrophilic pulmonary disease, comprising *Micrococcus luteus*-derived vesicles as an active ingredient.

In addition, an object of the present invention is to provide a food composition for preventing or alleviating a neutrophilic pulmonary disease, comprising *Micrococcus luteus-*derived vesicles as an active ingredient.

Furthermore, an object of the present invention is to provide an inhalant composition for preventing or treating a neutrophilic pulmonary disease, comprising *Micrococcus luteus*-derived vesicles as an active ingredient.

Further, an object of the present invention is to provide a quasi-drug composition for preventing or alleviating a neutrophilic pulmonary disease, comprising *Micrococcus luteus-*derived vesicles as an active ingredient.

In addition, an object of the present invention is to provide a composition for delivering a drug for treating a neutrophilic pulmonary disease, comprising *Micrococcus luteus*-derived vesicles as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention may provide a pharmaceutical composition for preventing or treating a neutrophilic pulmonary disease, comprising *Micrococcus luteus*-derived vesicles as an active ingredient.

In addition, the present invention may provide a food composition for preventing or alleviating a neutrophilic pulmonary disease, comprising *Micrococcus luteus-derived* vesicles as an active ingredient.

Furthermore, the present invention may provide an inhalant composition for preventing or treating a neutrophilic pulmonary disease, comprising *Micrococcus luteus-derived* vesicles as an active ingredient.

Further, the present invention may provide a quasi-drug composition for preventing or alleviating a neutrophilic pulmonary disease, comprising *Micrococcus luteus-derived* vesicles as an active ingredient.

In addition, the present invention may provide a composition for delivering a drug for treating a neutrophilic pulmonary disease, comprising *Micrococcus luteus-*derived vesicles as an active ingredient.

As an exemplary embodiment of the present invention, the pulmonary disease may be a pulmonary disease mediated by a nucleotide-binding oligomerization domain, leucine rich repeat and pyrin domain containing 3 (NLRP3) inflammasome, but is not limited thereto.

As another exemplary embodiment of the present invention, the pulmonary disease may be one or more diseases selected from the group consisting of asthma, emphysema, cystic fibrosis (CF), bacterial pneumonia, viral pneumonia, idiopathic pulmonary fibrosis (IPF), interstitial pneumonitis, acute respiratory distress syndrome (ARDS) and acute lung injury (ALI), and the asthma may be neutrophilic asthma, but is not limited thereto.

As still another exemplary embodiment of the present invention, the vesicles may have an average diameter of 10 to 200 nm, but is not limited thereto.

As yet another exemplary embodiment of the present invention, the vesicles may be naturally secreted or artificially produced from *Micrococcus luteus,* but are not limited thereto.

As yet another exemplary embodiment of the present invention, the composition may suppress the activity of the NLRP3 inflammasome, but is not limited thereto.

Further, the present invention provides a method for preventing or treating a neutrophilic pulmonary disease, the method comprising administering a composition comprising *Micrococcus luteus*-derived vesicles as an active ingredient to a subject in need thereof.

In addition, the present invention provides a use of a composition comprising *Micrococcus luteus-derived* vesicles as an active ingredient for preventing or treating a neutrophilic pulmonary disease.

Furthermore, the present invention provides a use of *Micrococcus luteus-*derived vesicles for preparing a drug for treating a neutrophilic pulmonary disease.

Further, the present invention provides a method for delivering a drug for treating a neutrophilic pulmonary disease, the method comprising administering a composition comprising *Micrococcus luteus-derived* vesicles, which carry a drug for treating a neutrophilic pulmonary disease to be targeted, as an active ingredient to a subject in need thereof.

In addition, the present invention provides a use of a composition comprising *Micrococcus luteus-derived* vesicles as an active ingredient for delivering a drug for treating a neutrophilic pulmonary disease.

### [Advantageous Effects]

The present inventors confirmed that when *Micrococcus luteus-derived* vesicles were orally or intranasally administered, the vesicles passed through the mucous membrane and were delivered to the lungs. Furthermore, the present inventors confirmed that not only the secretion of inflammatory mediators by biological causative factors was significantly suppressed when epithelial cells and inflammatory cells were treated with the vesicles, but also the secretion of NLRP3 protein by the biological causative factors was remarkably suppressed when the cells were treated with the vesicles. Further, it was confirmed that when the vesicles were administered to a mouse model of neutrophilic disease caused by biological causative factors, neutrophilic pulmonary inflammation and functional changes were significantly suppressed, so that *Micrococcus luteus-derived* vesicles according to the present invention will be able to be usefully used not only for developing a pharmaceutical or health functional food for preventing, ameliorating symptoms, or treating a neutrophilic pulmonary disease, but also as a drug delivery system for treating the disease.

### [Description of Drawings]

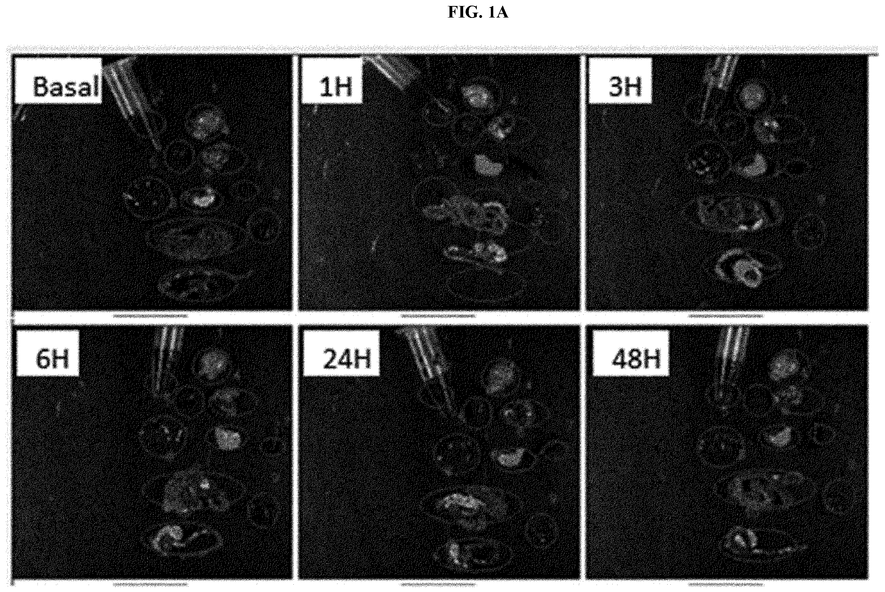

FIG. 1A is a view illustrating the intensity of fluorescence in each organ by orally administering *Micrococcus luteus-derived* vesicles to mice and then removing each organ over time.

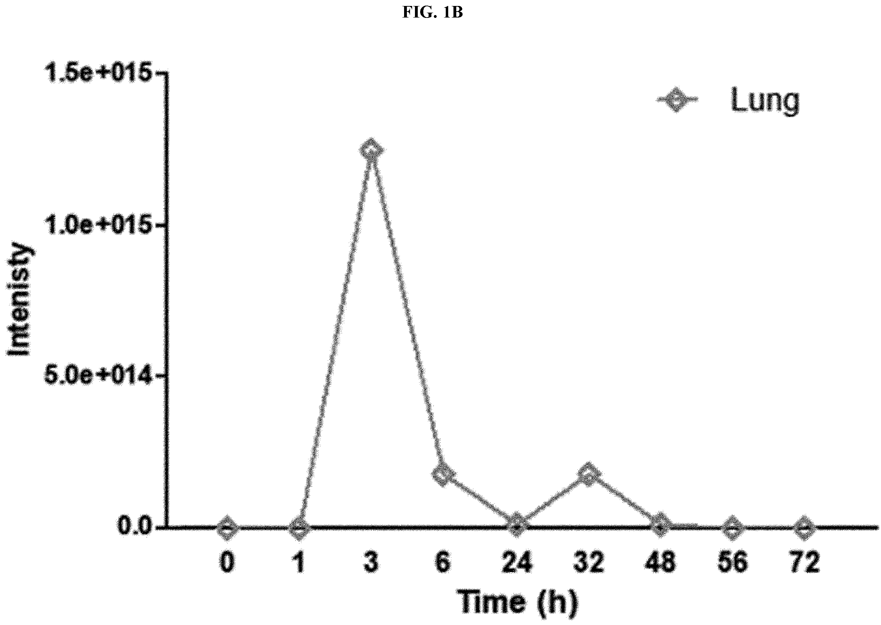

FIG. 1B is a view illustrating a pattern in which vesicles are distributed in the lungs over time after *Micrococcus luteus-derived* vesicles are orally administered to mice.

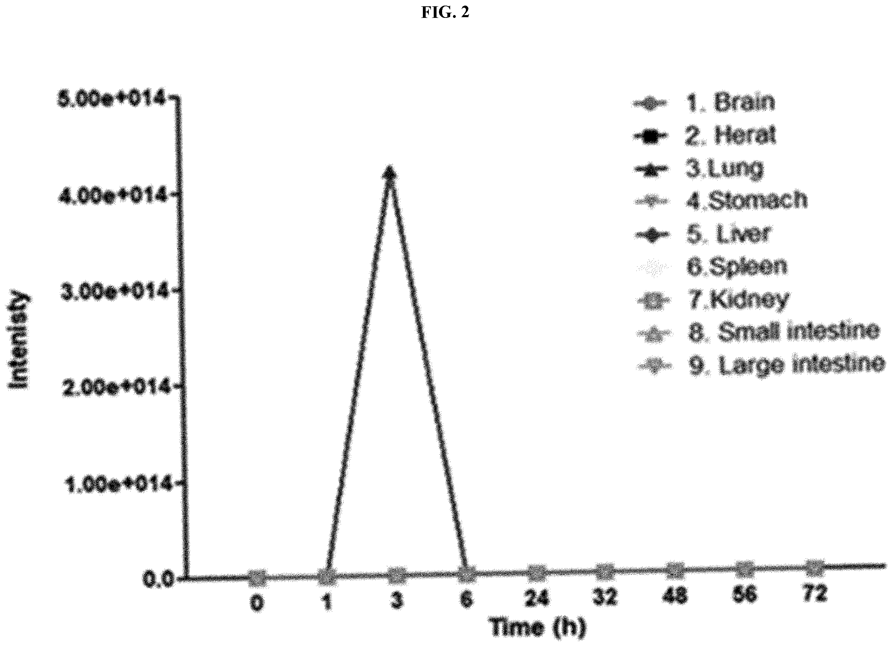

FIG. 2 is a view illustrating a pattern in which vesicles are distributed in organs over time after *Micrococcus luteus-derived* vesicles are intranasally administered to mice.

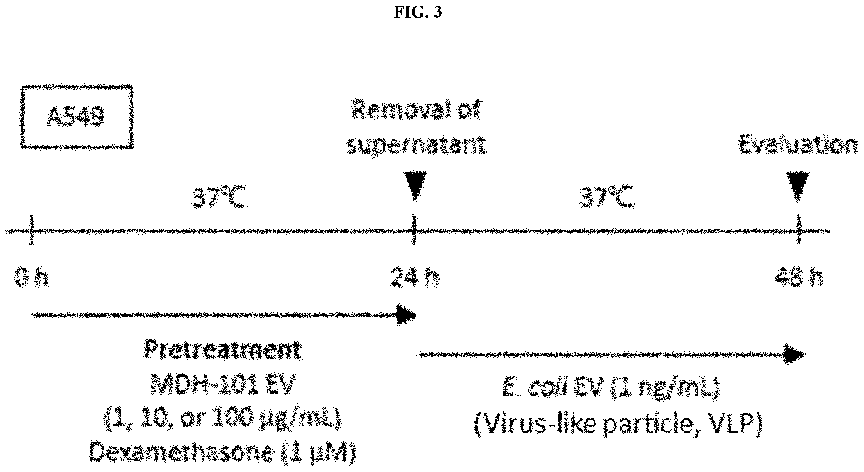

FIG. 3 is a view illustrating an experimental protocol for evaluating the inhibitory effect of *Micrococcus luteus-derived* vesicles (MDH-101EVs) on the secretion of inflammatory mediators from epithelial cells (A549).

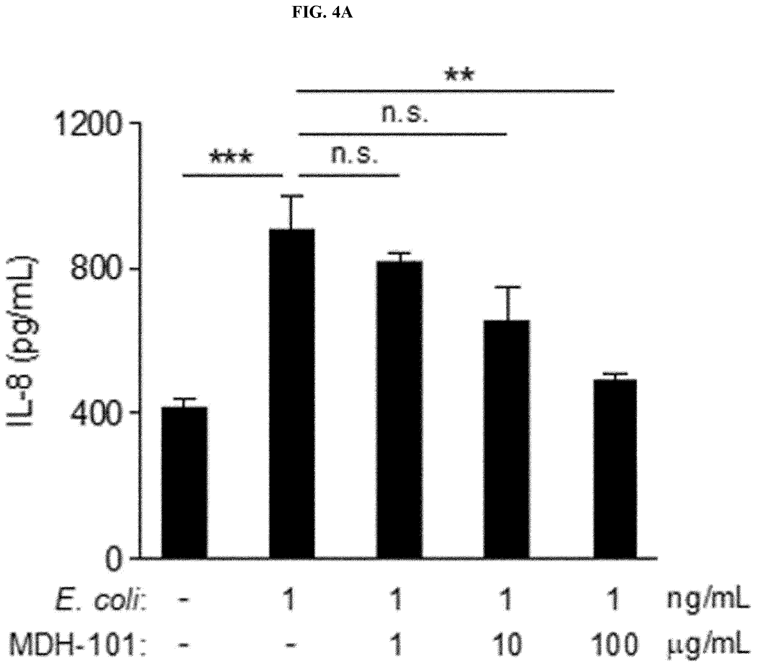

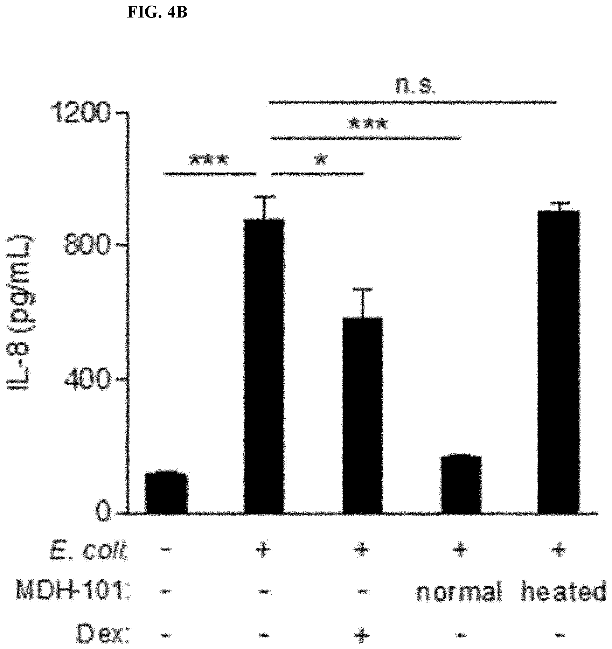

FIGS. 4A and 4B illustrate the results of confirming the inhibitory effect on the secretion of an inflammatory mediator IL-8 from epithelial cells by *Micrococcus luteus*-derived vesicles, FIG. 4A is a view illustrating the inhibitory effect on IL-8 secretion according to the concentration of *Micrococcus luteus-derived* vesicles (MDH-101), and FIG. 4B is a view illustrating the results of comparing the IL-8 secretion inhibitory effect of a positive control drug dexamethasone (Dex) and the vesicles (*P<0.05, **P<0.01, ***P<0.001, n.s. means not significant, and hereinafter the same).

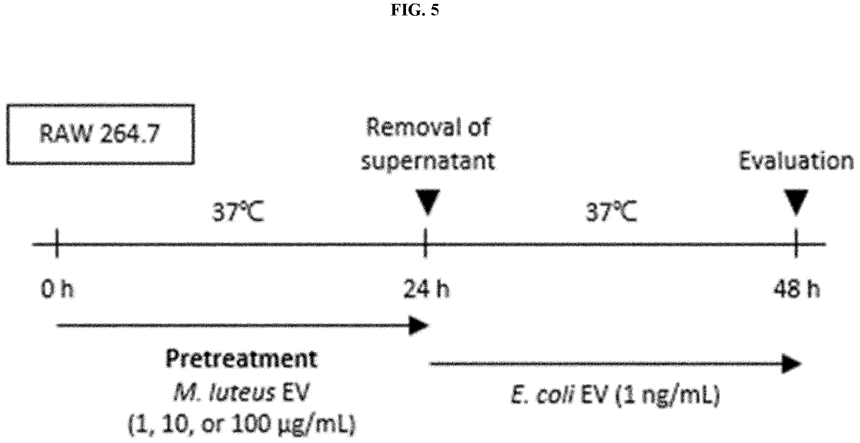

FIG. 5 is a view illustrating an experimental protocol for evaluating the inhibitory effect of *Micrococcus luteus-derived* vesicles (*Mluteus* EVs) on the secretion of inflammatory mediators from macrophages (RAW 264.7), which are inflammatory cells.

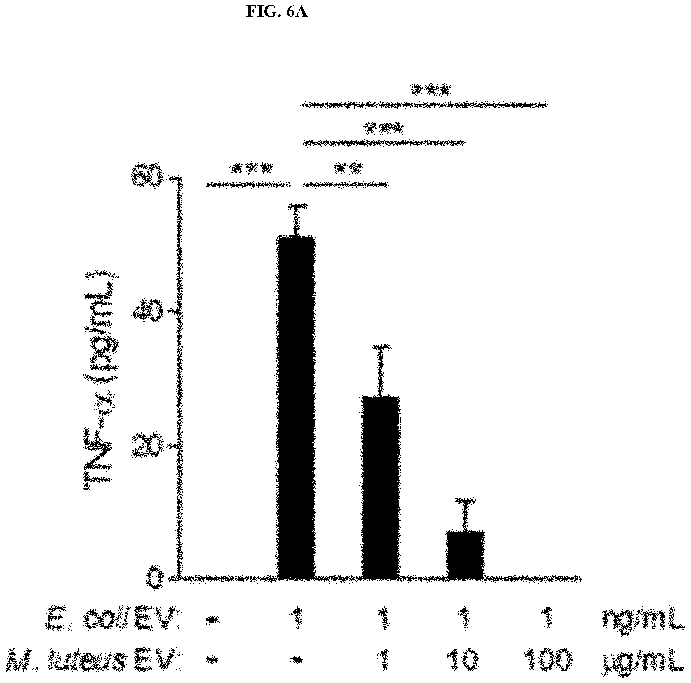

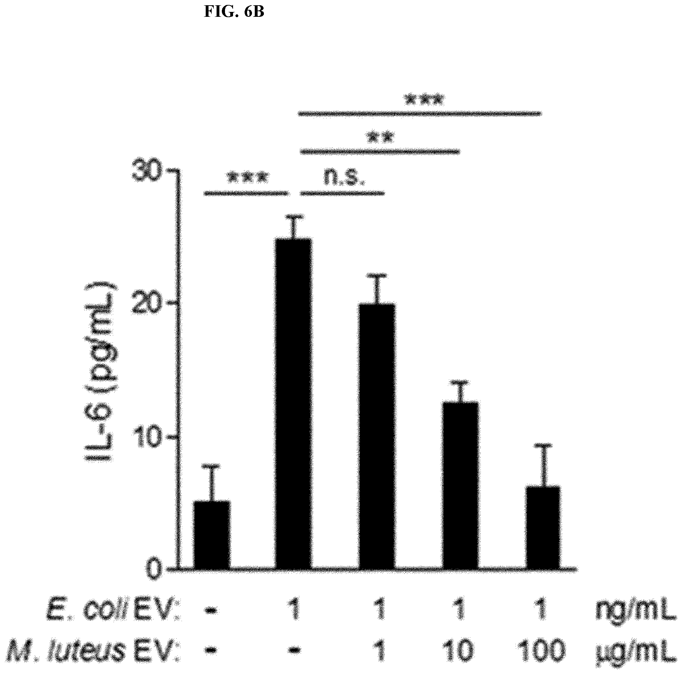

FIGS. 6A and 6B confirm the inhibitory effect of *Micrococcus luteus-derived* vesicles (*Mluteus* EVs) on the secretion of inflammatory mediators from macrophages, FIG. 6A is a view illustrating the inhibitory effect on TNF-α secretion, and FIG. 6B is a view illustrating the inhibitory effect on IL-6 secretion.

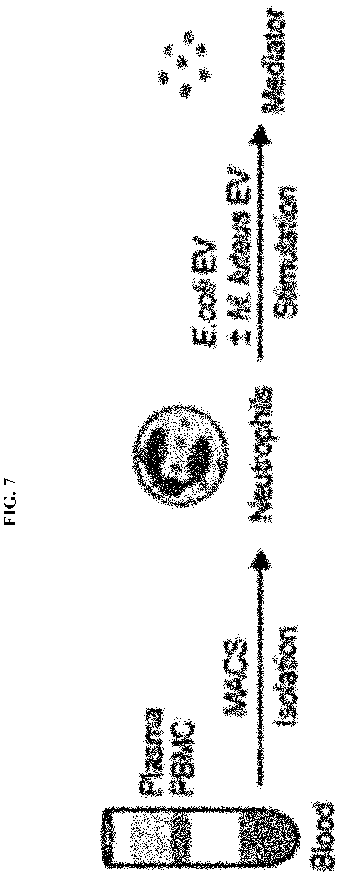

FIG. 7 is a view illustrating an experimental protocol for evaluating the effect of suppressing the secretion of an inflammatory mediator by E. coli-derived vesicles *(E. coli* EVs), which are an inflammatory causative factor, by treating neutrophils isolated from peripheral blood with *Micrococcus luteus-derived* vesicles *(M. luteus* EVs) or a positive control drug dexamethasone (Dex) (plasma PBMCs: plasma peripheral blood mononuclear cells).

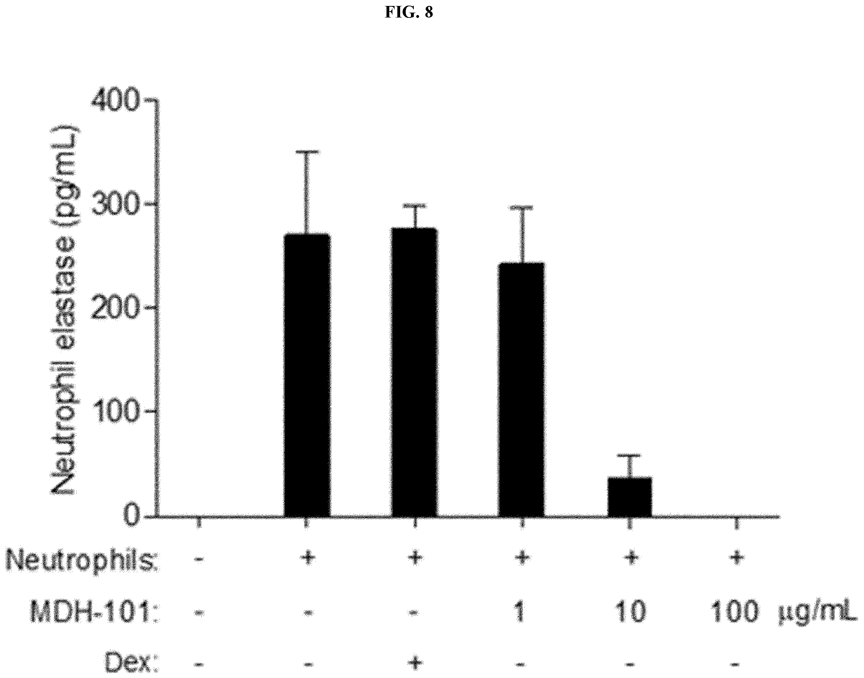

FIG. 8 is a view illustrating the results of evaluating the degree of activation of neutrophils by *E*. *coli*-derived vesicles *(E. coli* EVs) by neutrophil elastase (NE) secretion after treating neutrophils isolated from peripheral blood with *Micrococcus luteus-derived* vesicles (MDH-101) or a positive control drug dexamethasone.

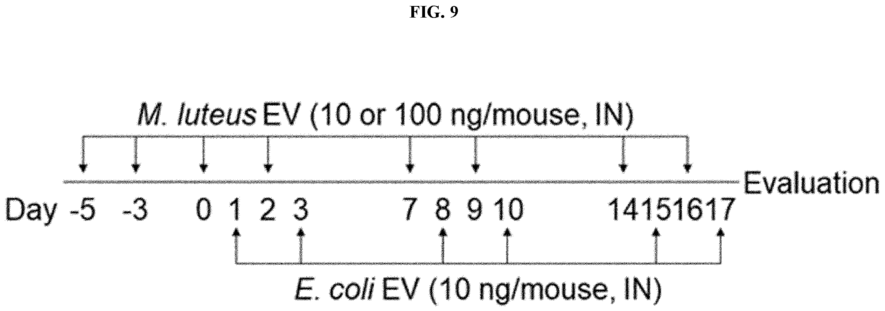

FIG. 9 is an experimental protocol for evaluating the anti-inflammatory effect of *Micrococcus luteus-derived* vesicles *(M. luteus* EVs) in a mouse model of neutrophilic pulmonary disease caused by *E*. *coli*-derived vesicles *(E. coli* EVs) which are pathogenic nanoparticles.

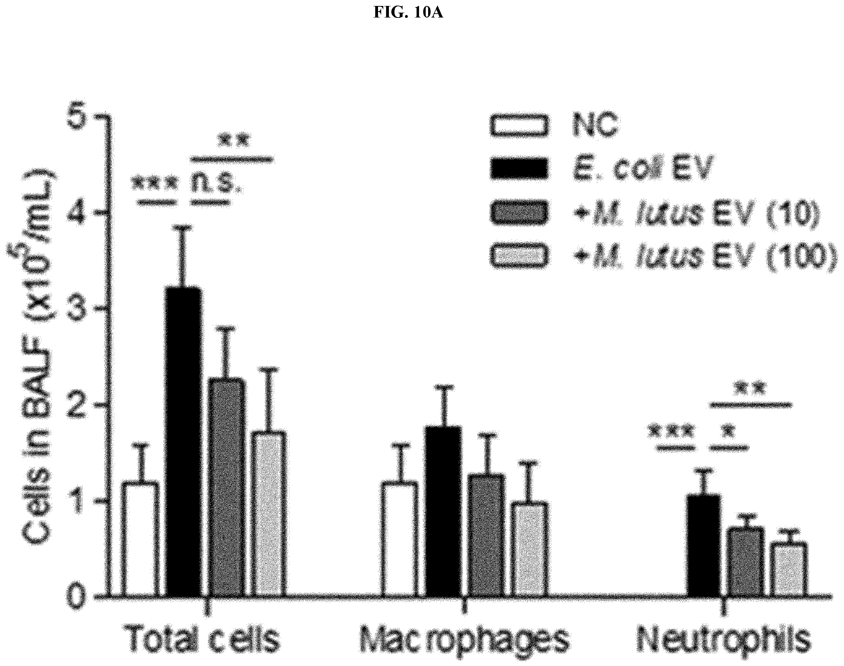

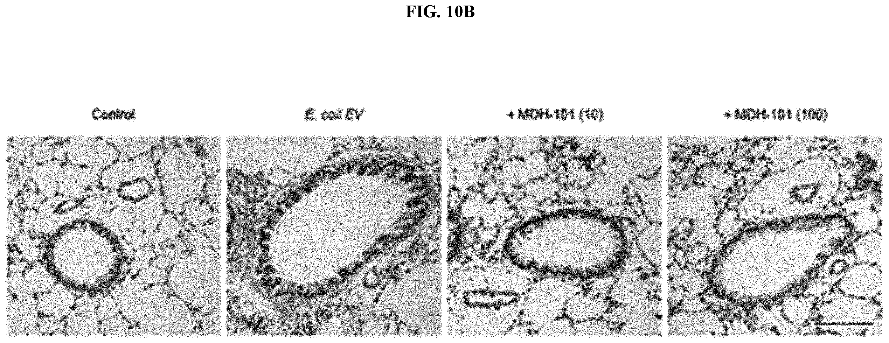

FIGS. 10A and 10B illustrate the results of confirming the anti-inflammatory effect by *Micrococcus luteus-derived* vesicles *(M. luteus* EVs) in a mouse model of neutrophilic pulmonary disease caused by *E*. *coli*-derived vesicles *(E. coli* EVs) which are pathogenic nanoparticles, FIG. 10A is a view illustrating the results of confirming the number of macrophages and neutrophils in bronchoalveolar lavage fluid (BALF) of a mouse model (NC: negative control), and FIG. 10B is a view illustrating the results of confirming inflammatory cell infiltration in pulmonary tissue.

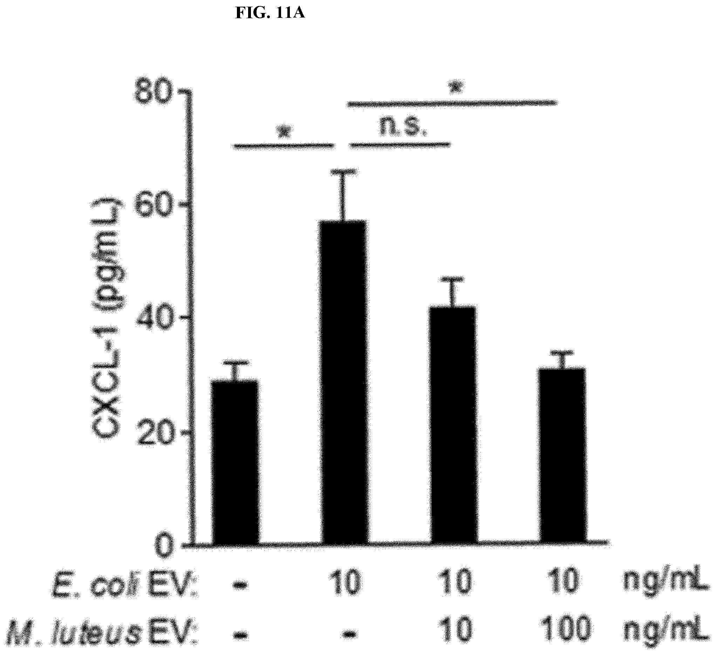

FIGS. 11A to 11C confirm the inhibitory effect on inflammatory mediator secretion by *Micrococcus luteus-derived* vesicles *(M. luteus* EVs) in a mouse model of neutrophilic pulmonary disease caused by *E*. *coli*-derived vesicles *(E. coli* EVs) which are pathogenic nanoparticles, and are views illustrating the results of confirming the inhibitory effect on CXCL-1 (a), TNF-α (b), and IL-1β (c) secretion in bronchoalveolar lavage fluid (BALF) of mice.

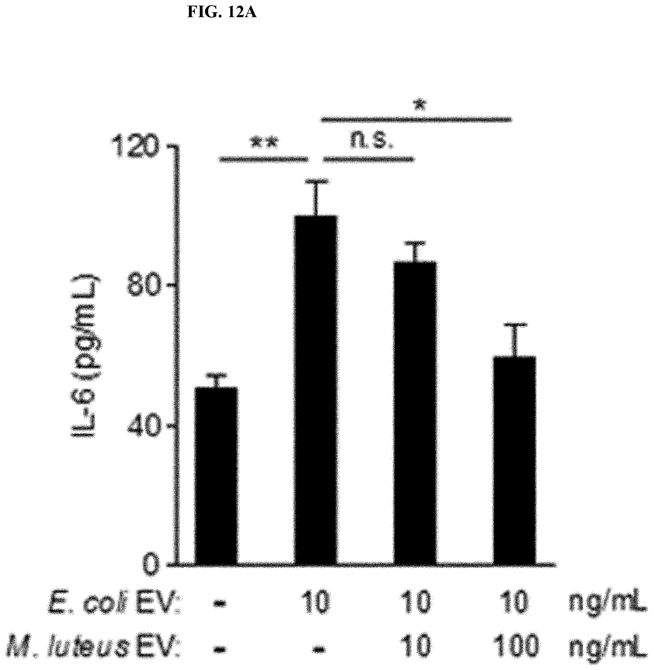

FIGS. 12A to 12C confirm the effect of *Micrococcus luteus-derived* vesicles *(M. luteus* EVs) on the secretion of immune-related cytokines in a mouse model of neutrophilic pulmonary disease caused by *E. coli*-derived vesicles *(E. coli* EVs) which are pathogenic nanoparticles, and are views illustrating the results of confirming the secretion of IL-6, IL-17, and IL-10, respectively.

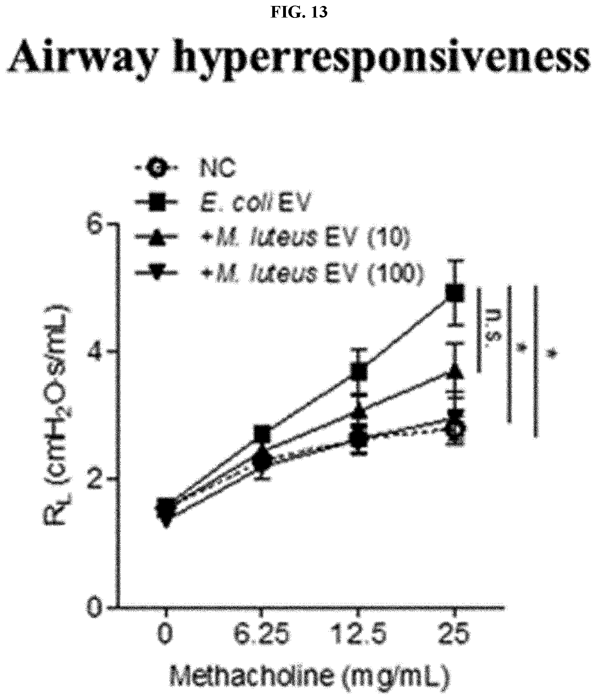

FIG. 13 is a view illustrating the effect of *Micrococcus luteus-derived* vesicles *(M. luteus* EVs) on airway hyperresponsiveness in a mouse model of neutrophilic pulmonary disease caused by *E. coli-*derived vesicles *(E. coli* EVs) which are pathogenic nanoparticles (NC: negative control).

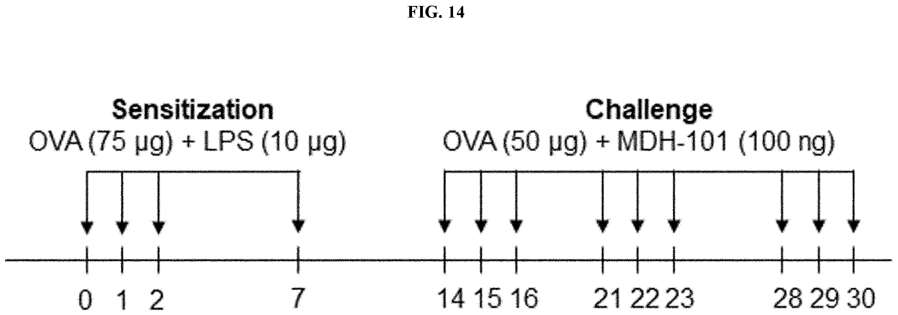

FIG. 14 is an experimental protocol for evaluating the anti-inflammatory effect of *Micrococcus luteus-derived* vesicles (MDH-101) in a mouse model of neutrophilic pulmonary disease caused by allergens contaminated with LPS (LPS + ovalbumin (OVA)).

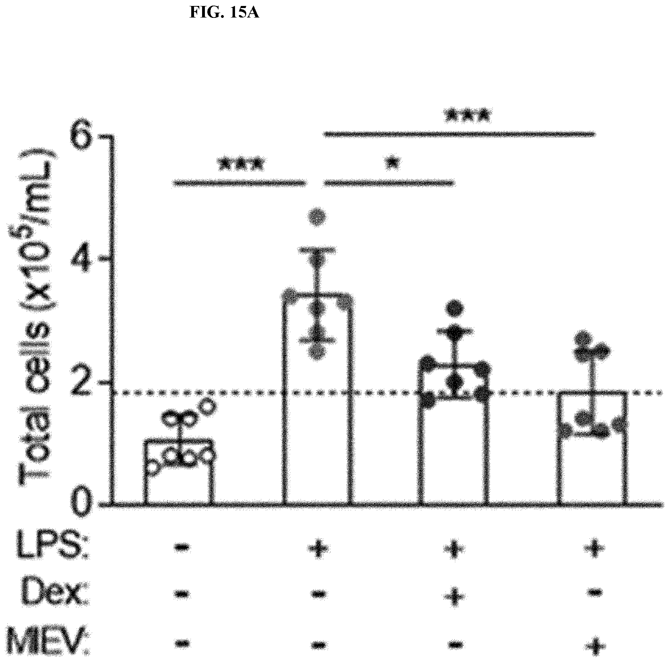

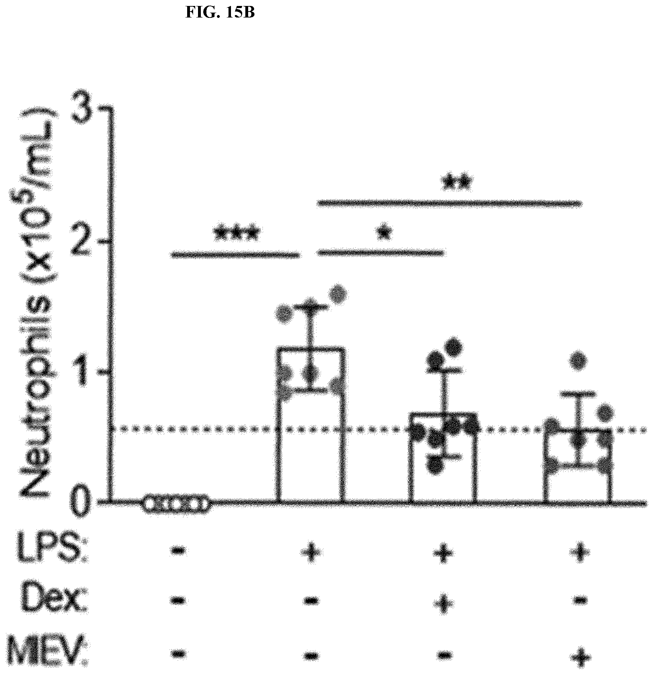

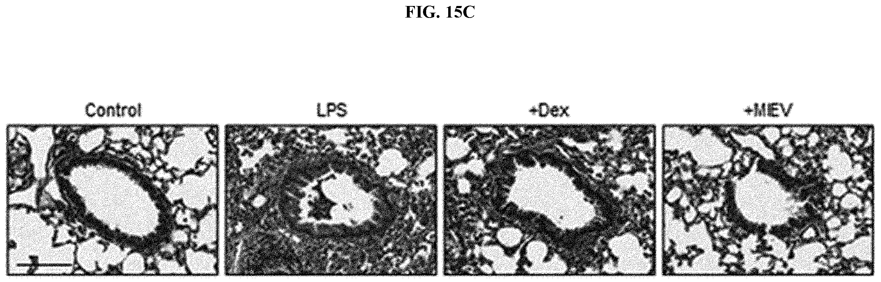

FIGS. 15A to 15C confirm the anti-inflammatory effect of *Micrococcus luteus*-derived vesicles (MlEVs) in a mouse model of neutrophilic pulmonary disease caused by allergens contaminated with LPS (LPS + OVA), FIGS. 15A and 15B are views illustrating the results of confirming the total number of inflammatory cells and the number of neutrophils in bronchoalveolar lavage fluid (BALF), and FIG. 15C is a view illustrating the results of confirming inflammatory cell infiltration in pulmonary tissue (LPS: allergens contaminated with LPS).

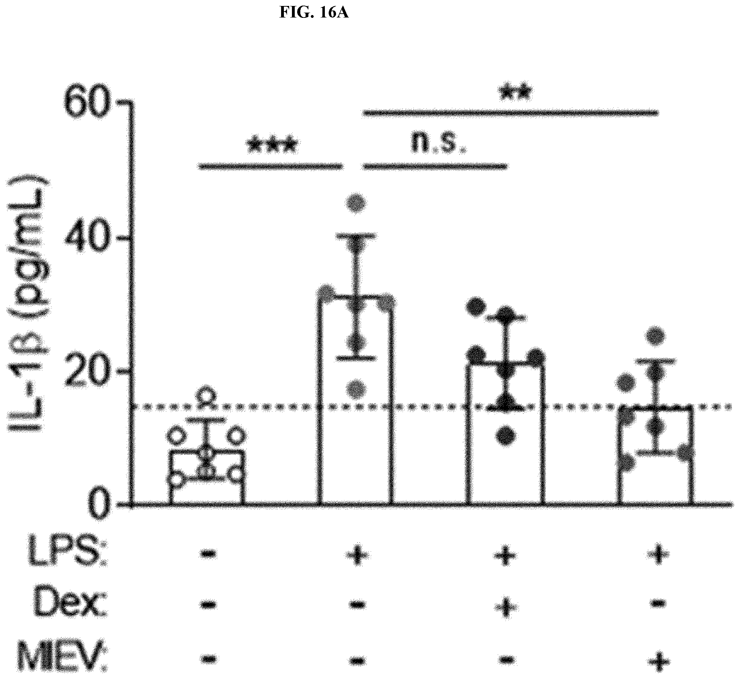

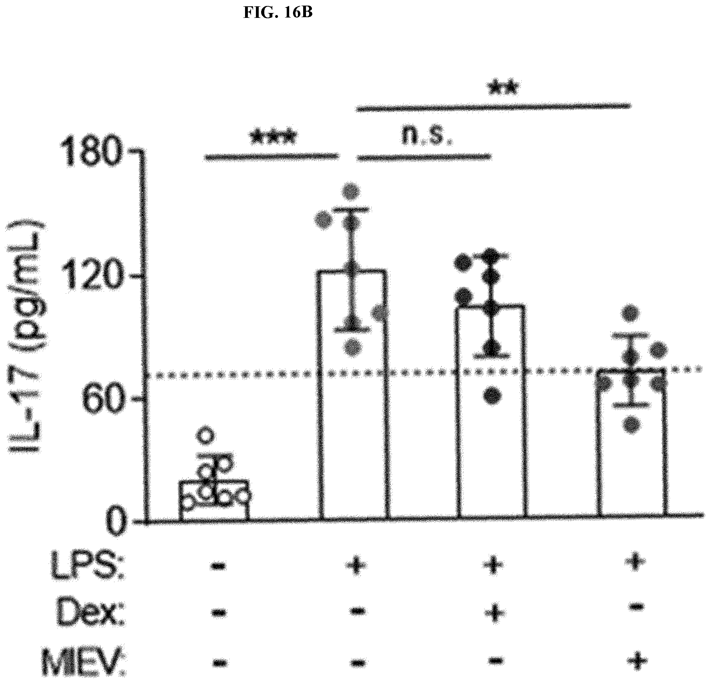

FIGS. 16A and 16B views confirming the effect of *Micrococcus luteus-*derived vesicles (MlEVs) on an inflammatory mediator IL-1β (A) and a Th17 immune response indicator IL-17 (b) in a mouse model of neutrophilic pulmonary disease caused by allergens contaminated with LPS (LPS + OVA) (LPS: allergens contaminated with LPS).

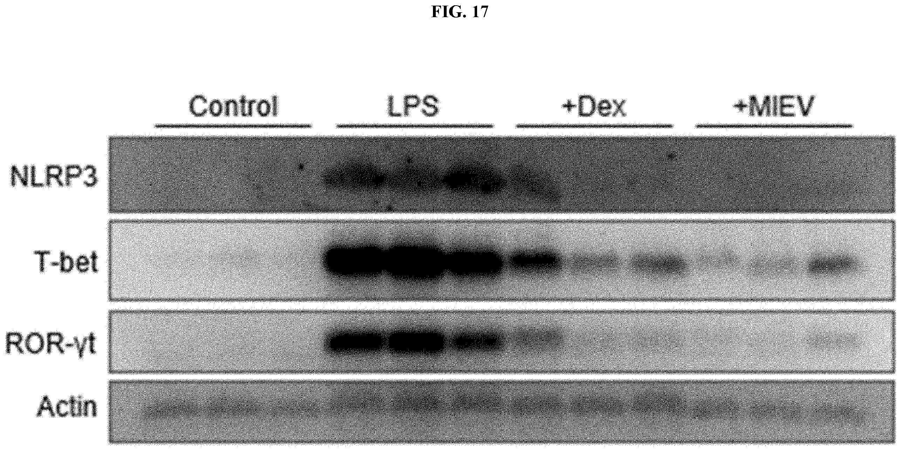

FIG. 17 is a view illustrating the results of confirming the expression of NLRP3, T-bet and ROR-γt proteins, which are immune function regulatory proteins, in mouse pulmonary tissue to evaluate the immune function regulatory effect of *Micrococcus luteus-derived* vesicles (MlEVs) in a mouse model of neutrophilic pulmonary disease caused by allergens contaminated with LPS (LPS: allergens contaminated with LPS).

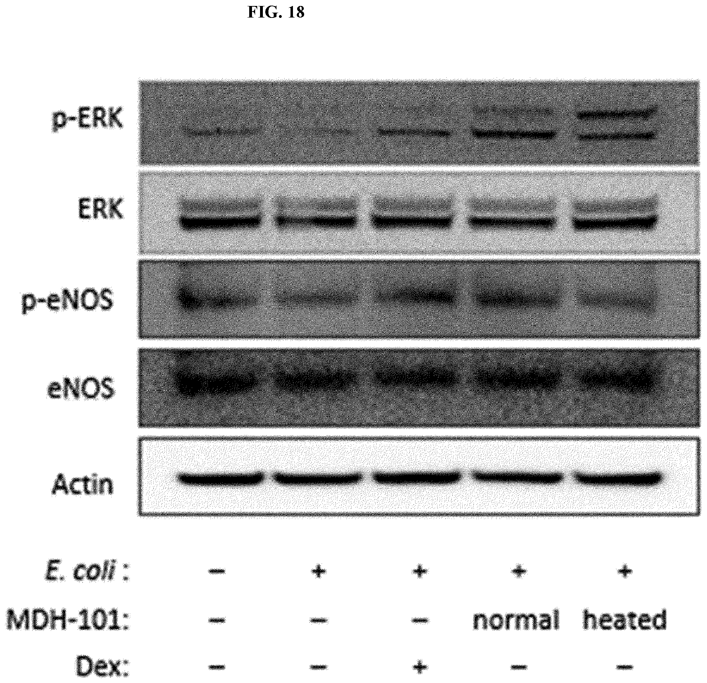

FIG. 18 is a view illustrating the results of evaluating the effect on eNOS signaling activity by *E. coli-*derived vesicles, which are an inflammatory causative factor, after treating pulmonary epithelial cells with *Micrococcus luteus-derived* vesicles or a positive control drug dexamethasone (Dex), in order to evaluate the effect of *Micrococcus luteus-derived* vesicles (MDH-101) on eNOS signals, which induce the production of nitric oxide (NO) which suppresses airway hyperresponsiveness.

### [Best Mode]

The present invention relates to vesicles derived from *Micrococcus luteus* and a use thereof.

Hereinafter, the present invention will be described in detail.

The present inventors confirmed that when *Micrococcus luteus-derived* vesicles were orally or intranasally administered, the vesicles passed through the mucous membrane and were distributed in pulmonary tissue. Further, the present inventors confirmed that not only the secretion of inflammatory mediators were remarkably suppressed by biological causative factors when epithelial cells were treated with the vesicles, but also the secretion of inflammatory mediators and the activation of neutrophils caused by biological causative factors were suppressed in a dose-dependent manner when macrophages and neutrophils, which are representative inflammatory cells, were treated with the vesicles. In addition, the present inventors confirmed that the expression of NLRP3 protein, which is expressed by biological causative factors, was remarkably suppressed when the vesicles were administered, and confirmed that the eNOS signals, which are suppressed by biological causative factors, were significantly restored when the vesicles were administered. Furthermore, the present inventors confirmed that when the vesicles were administered to a mouse model of neutrophilic pulmonary disease caused by pathogenic nanoparticles and LPS-contaminated allergens, the development of neutrophilic pulmonary inflammation, immune dysfunction and airway hyperresponsiveness was significantly suppressed, thereby completing the present invention based on this.

Therefore, the present invention may provide a pharmaceutical composition for preventing or treating a neutrophilic pulmonary disease, comprising *Micrococcus luteus*-derived vesicles as an active ingredient.

As used herein, the term "neutrophilic pulmonary disease" refers to a disease characterized by neutrophilic infiltration in pulmonary tissue, which is caused by biological causative factors present in dust such as viruses, bacterial-derived vesicles, lipopolysaccharide (LPS), and allergens, and in the present invention, specifically, the neutrophilic pulmonary disease may be a pulmonary disease mediated by a nucleotide-binding oligomerization domain, leucine rich repeat and pyrin domain containing 3 (NLRP3) inflammasome, but is not limited thereto, and may include any pulmonary disease characterized by neutrophilic infiltration in pulmonary tissue.

Examples of the neutrophilic pulmonary disease include asthma, emphysema, cystic fibrosis (CF), bacterial pneumonia, viral pneumonia, idiopathic pulmonary fibrosis (IPF), interstitial pneumonitis, acute respiratory distress syndrome (ARDS) and acute lung injury (ALI), but are not limited thereto.

The "pulmonary disease mediated by the NLRP3 inflammasome" refers to a pulmonary disease caused by abnormally excessive formation of an inflammasome, and in the present invention, since the *Micrococcus luteus-derived* vesicles can suppress the formation of the NLRP3 inflammasome, neutrophilic pulmonary diseases mediated by the NLRP3 inflammasome may be effectively prevented, ameliorated, or treated by the *Micrococcus luteus-derived* vesicles.

Further, the "asthma" refers to a representative allergic disease caused by a combination of genetic and environmental factors in a state in which the respiratory tract in the lungs become very sensitive, may be divided into eosinophilic and neutrophilic asthma according to the type and infiltration pattern of immune cells that penetrate the airway or pulmonary tissue, and in the present invention, specifically, the asthma may be neutrophilic asthma.

As used herein, the term "extracellular vesicle" or "vesicle" refers to a structure formed of a nano-sized membrane secreted from various bacteria, and examples thereof include vesicles derived from gram-negative bacteria such as *E. coli* containing lipopolysaccharides, toxic proteins and bacterial DNA and RNA, and the like, outer membrane vesicles (OMVs), vesicles derived from gram-positive bacteria such as *Micrococcus* bacteria containing peptidoglycan and lipoteichoic acid, which are bacterial cell wall components, and the like in addition to proteins and nucleic acids, and the like.

In the present invention, the extracellular vesicles or vesicles may collectively refer to all structures which are naturally secreted from *Micrococcus luteus* or consist of artificially produced membranes, and may be variously represented by MDH-101, MDH-101 EV, *M. luteus* EV or MlEV in the present invention.

The vesicles may be isolated by heat treatment or autoclaving during *Micrococcus luteus* culture, or using one or more methods selected from the group consisting of centrifugation, ultracentrifugation, autoclaving, extrusion, sonication, cell lysis, homogenization, freezing-thawing, electroporation, mechanical degradation, chemical treatment, filtration with a filter, gel filtration chromatography, pre-flow electrophoresis, and capillary electrophoresis of the cell culture. In addition, for isolation, washing for removing impurities, and concentration of the obtained vesicles may be further performed.

In the present invention , the vesicles isolated by the method are in the form of spheres, and may have an average diameter of 10 to 200 nm, 10 to 190 nm, 10 to 180 nm, 10 to 170 nm, 10 to 160 nm, 10 to 150 nm, 10 to 140 nm, 10 to 130 nm, 10 to 120 nm, 10 to 110 nm, 10 to 100 nm, 10 to 90 nm, 10 to 80 nm, 10 to 70 nm, 10 to 60 nm, 10 to 50 nm, 20 to 200 nm, 20 to 180 nm, 20 to 160 nm, 20 to 140 nm, 20 to 120 nm, 20 to 100 nm, or 20 to 80 nm, preferably 20 to 200 nm, but the average diameter is not limited thereto.

As used herein, the term "comprising as an active ingredient" refers to including a sufficient amount to achieve the efficacy or activity of the *Micrococcus luteus-derived* vesicles.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition of the present invention may include a pharmaceutically acceptable carrier. The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the composition may be formulated into an injectable formulation, such as an aqueous solution, a suspension, and an emulsion, a pill, a capsule, a granule, or a tablet by additionally adding a diluent, a dispersant, a surfactant, a binder, a lubricant, and the like. With regard to suitable pharmaceutically acceptable carriers and formulations, the composition may be preferably formulated according to each ingredient by using the method disclosed in the Remington's literature. The pharmaceutical composition of the present invention is not particularly limited in formulation, but may be formulated into an injection, an inhalant, an external preparation for skin, an oral ingestion, or the like.

The pharmaceutical composition of the present invention may be orally administered or may be parenterally administered (for example, administered intravenously, subcutaneously, intradermally, intranasally or intratracheally) according to the target method, and the administration dose may vary depending on the patient's condition and body weight, severity of disease, drug form, and administration route and period, but may be appropriately selected by those of ordinary skill in the art.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

Specifically, the effective amount of the pharmaceutical composition according to the present invention may vary depending on the patient's age, sex, and body weight, and generally, 0.001 to 150 mg of the composition and preferably, 0.01 to 100 mg of the composition, per 1 kg of body weight, may be administered daily or every other day or administered one to three times a day. However, since the effective amount may be increased or decreased depending on the administration route, the severity of obesity, gender, body weight, age, and the like, the dosage is not intended to limit the scope of the present invention in any way.

As used herein, the term "prevention" refers to all actions that suppress a neutrophilic pulmonary disease such as asthma, emphysema, cystic fibrosis (CF), bacterial pneumonia, viral pneumonia, idiopathic pulmonary fibrosis (IPF), interstitial pneumonitis, acute respiratory distress syndrome (ARDS) and acute lung injury (ALI), or delays the development thereof by administering the composition according to the present invention.

As used herein, the term "treatment" refers to all actions that ameliorate or beneficially change the symptoms of a neutrophilic pulmonary disease such as asthma, emphysema, cystic fibrosis (CF), bacterial pneumonia, viral pneumonia, idiopathic pulmonary fibrosis (IPF), interstitial pneumonitis, acute respiratory distress syndrome (ARDS) and acute lung injury (ALI) by administering the composition according to the present invention.

Further, the present invention provides a method for preventing or treating a neutrophilic pulmonary disease, the method comprising administering a composition comprising *Micrococcus luteus*-derived vesicles as an active ingredient to a subject in need thereof.

In addition, the present invention provides a use of a composition comprising *Micrococcus luteus-derived* vesicles as an active ingredient for preventing or treating a neutrophilic pulmonary disease.

Furthermore, the present invention provides a use of *Micrococcus luteus-*derived vesicles for preparing a drug for treating a neutrophilic pulmonary disease.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

In addition, the present invention may provide a food composition for preventing or alleviating a neutrophilic pulmonary disease, comprising *Micrococcus luteus-derived* vesicles as an active ingredient.

As used herein, term "alleviation" refers to all actions that at least reduce a parameter associated with a condition to be treated, for example, the degree of symptoms.

The food composition may be a health functional food composition, but is not limited thereto.

The vesicles according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be below the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

Furthermore, the present invention may provide an inhalant composition for preventing or treating a neutrophilic pulmonary disease, comprising *Micrococcus luteus*-derived vesicles as an active ingredient.

In the case of an inhalant composition, the compound may be formulated according to a method known in the art, and may be conveniently delivered in the form of an aerosol spray from a pressurized pack or a nebulizer by using a suitable propellant, for example, dichlorofluoromethane, trichlorofluoromethane, dichlorotetrafluoroethane, carbon dioxide, or other suitable gases. In the case of the pressurized aerosol, a dosage unit may be determined by providing a valve for transferring a metered amount. For example, a gelatin capsule and a cartridge for use in an inhaler or insufflator may be formulated so as to contain a powder mixture of a compound and a suitable powder base such as lactose or starch.

Further, the present invention may provide a quasi-drug composition for preventing or alleviating a neutrophilic pulmonary disease, comprising *Micrococcus luteus-derived* vesicles as an active ingredient.

When the *Micrococcus luteus-derived* vesicles are used as a quasi-drug additive, the vesicles may be added as they are or used with another quasi-drug or other quasi-drug ingredients, and may be appropriately used according to a typical method. The amount of active ingredient mixed may be suitably determined according to the purpose of use (prevention, health or therapeutic treatment). The quasi-drug composition is not particularly limited in its formulation, and may be variously formulated in the form of quasi-drugs known in the art, which are effective for preventing or ameliorating a neutrophilic pulmonary disease. The quasi-drug composition may be, for example, an antiseptic cleanser, shower foam, mouthwash, wet wipes, detergent soap, hand wash, a humidifier filler, a mask, ointment, inhalant or a filter filler, but is not limited thereto.

In addition, the present invention may provide a composition for delivering a drug for treating a neutrophilic pulmonary disease, comprising *Micrococcus luteus-*derived vesicles as an active ingredient.

As used herein, the term "drug delivery" refers to all means or actions which load and deliver a drug in the composition according to the present invention in order to deliver the drug to a specific organ, tissue, cell, or organelle.

Further, the present invention provides a method for delivering a drug for treating a neutrophilic pulmonary disease, the method comprising administering a composition comprising *Micrococcus luteus-derived* vesicles, which carries a drug for treating a neutrophilic pulmonary disease to be targeted, as an active ingredient to a subject in need thereof.

In addition, the present invention provides a use of a composition comprising *Micrococcus luteus-derived* vesicles as an active ingredient for delivering a drug for treating a neutrophilic pulmonary disease.

Hereinafter, preferred Examples for helping the understanding of the present invention will be suggested. However, the following Examples are provided only to more easily understand the present invention, and the contents of the present invention are not limited by the following Examples.

### [Modes of the Invention]

### [Examples]

### Example 1. Isolation of vesicles from Micrococcus luteus culture fluid

After culturing a *Micrococcus luteus* strain, vesicles thereof were isolated, analyzed and characterized. *Micrococcus luteus* was cultured in a de Man-Rogosa and Sharpe (MRS) medium until absorbance (OD 600) became 1.0 to 1.5 in a 37 °C aerobic chamber, and then sub-cultured. Subsequently, the medium supernatant containing the strain was recovered, centrifuged at 10,000 g and 4 °C for 20 minutes, and the strain was removed and then filtered through a 0.22-µm filter. And the filtered supernatant was concentrated to a volume of 50 mL using a 100 kDa Pellicon 2 Cassette filter membrane (Merck Millipore, US) and a MasterFlex pump system (Cole-Parmer, US) through microfiltration. Then, the concentrated supernatant was filtered again using a 0.22 µm filter. Subsequently, the protein was quantified using a BCA assay, and the following experiments were performed on the obtained vesicles.

### Example 2. Analysis of pharmacokinetic characteristics during oral administration of Micrococcus luteus-derived vesicles

In order to investigate the pharmacokinetic characteristics of *Micrococcus luteus*-derived vesicles during oral administration, the fluorescence expressed in each organ was measured for up to 48 hours by orally administering *Micrococcus luteus-*derived vesicles stained with a fluorescent staining reagent to mice. As illustrated in FIG. 1A, it was confirmed that when the organ distribution of fluorescently-stained *Micrococcus luteus-derived* vesicles was evaluated by images over time, the vesicles were distributed in various organs. In addition, as illustrated in FIG. 1B, it was confirmed that when the fluorescence intensity of *Micrococcus luteus-derived* vesicles expressed in the lungs was illustrated by a graph, the vesicles were distributed in the lungs from 3 hours after oral administration, this continued until 24 hours, and after that, most of the fluorescence signals had disappeared. From the above results, it can be seen that *Micrococcus luteus*-derived vesicles pass through the mucous membrane when orally administered, are absorbed into the body, move to the lungs, and are distributed.

### Example 3. Analysis of pharmacokinetic characteristics during inhalation administration of Micrococcus luteus-derived vesicles

In order to investigate the pharmacokinetic characteristics of *Micrococcus luteus*-derived vesicles during inhalation administration, the fluorescence expressed in each organ was measured for up to 72 hours by intranasally administering *Micrococcus luteus*-derived vesicles stained with a fluorescent staining reagent to mice. As illustrated in FIG. 2, it was confirmed that when the organ distribution of fluorescently-stained *Micrococcus luteus-derived* vesicles was image-evaluated over time, *Micrococcus luteus-derived* vesicles were mainly distributed in pulmonary tissue, appeared from 1 hour to 6 hours after administration, and was excreted thereafter. From the above results, it can be seen that *Micrococcus luteus-derived* vesicles pass through the mucous membrane when intranasally administered, are absorbed into the body, and are distributed in pulmonary tissue.

### Example 4. Evaluation of anti-inflammatory effects of Micrococcus luteus-derived vesicles in epithelial cells

As illustrated in FIG. 3, epithelial cells (A549 cells) were pre-treated with *Micrococcus luteus-derived* vesicles *(M. luteus* EVs) or a positive control drug dexamethasone (Dex), and then treated with *E. coli-*derived vesicles *(E. coli* EVs) to measure the secretion amount of an inflammatory cytokine IL-8 by enzyme-linked immunosorbent assay (ELISA, R&D Systems). Specifically, macrophages were pre-treated with the *Micrococcus luteus-derived* vesicles at various concentrations (1, 10, and 100 µg/mL) for 24 hours, and then treated with *E. coli-*derived vesicles at a concentration of 1 ng/mL for 24 hours to measure IL-8 secreted into the medium.

As a result, as illustrated in FIG. 4A, it was confirmed that the secretion of IL-8 was suppressed by *Micrococcus luteus*-derived vesicles in a dose-dependent manner. Further, as illustrated in FIG. 4B, it was confirmed that when compared with the control drug dexamethasone, the inhibitory effect on the secretion of IL-8, which is a representative cytokine that induces neutrophil infiltration, was even better, and when the vesicles were heat-treated and then administered, the IL-8 secretion inhibitory effect was lost. From the above results, it can be seen that *Micrococcus luteus-derived* vesicles have better therapeutic efficacy against neutrophilic inflammation than the representative anti-inflammatory drug dexamethasone, and from the fact that the anti-inflammatory effect by *Micrococcus luteus*-derived vesicles is lost upon heat treatment, it can be seen that the anti-inflammatory action is mediated by proteins in vesicles.

### Example 5. Evaluation of anti-inflammatory effects of Micrococcus luteus-derived vesicles in macrophages which are inflammatory cells

As illustrated in FIG. 5, after macrophages (RAW 264.7 cells) were pre-treated with *Micrococcus luteus-derived* vesicles *(M. luteus* EVs), and then treated with *E. coli-*derived vesicles *(E. coli* EVs) which induce inflammation, the secretion levels of inflammatory cytokines TNF-α and IL-6 were measured by ELISA (R&D Systems) method. Specifically, after macrophages were pre-treated with the *Micrococcus luteus-derived* vesicles at various concentrations (1, 10, and 100 µg/mL) for 24 hours, the secretion amounts of TNF-α and IL-6 secreted to the media were measured by treating the macrophages with *E. coli-*derived vesicles at a concentration of 1 ng/mL for 24 hours.

As a result, as illustrated in FIGS. 6A and 6B, it was confirmed that when macrophages were pre-treated with the *Micrococcus luteus-derived* vesicles, the secretion of TNF-α (FIG. 6A) and IL-6 (FIG. 6B) by the *E. coli-*derived vesicles was suppressed in a dose-dependent manner. From the above results, it can be seen that *Micrococcus luteus-derived* vesicles efficiently suppress the development of inflammation caused by pathogenic biological causative factors.

### Example 6. Confirmation of neutrophil activation inhibitory effect of Micrococcus luteus-derived vesicles in neutrophils, which are inflammatory cells

As illustrated in FIG. 7, after neutrophils were extracted from human blood using Lymphoprep to evaluate the activation of neutrophils, neutrophil elastase (NE), which is a granule protein in neutrophils, was measured by ELISA (R&D Systems). Specifically, after 10 mL of blood was collected in a tube including an ACD solution, the blood was carefully placed on 20 mL of Lymphoprep and centrifuged. After centrifugation, a red blood layer was collected and mixed with 12 mL of dextran, and the resulting mixture was allowed to stand at room temperature for 45 minutes. After layer separation, 40 mL of 1X HBSS was added to the supernatant and centrifuged once again, and then red blood cells were removed with autoclaved Millipore water, and neutrophils were isolated using a neutrophil isolation kit (MACS). Isolated neutrophils were cultured using an RPMI1640 medium, and co-treated with *E. coli*-derived vesicles *(E. coli* EVs) and *Micrococcus luteus-derived* vesicles *(M. luteus* EVs) for 24 hours, and then NE was measured in the medium. The isolated neutrophils were treated with dexamethasone (Dex) as a positive control drug.

As a result, as illustrated in FIG. 8, the control drug dexamethasone could not suppress the secretion of neutrophil elastase (NE), which is an indicator of neutrophilic activation, but when neutrophils were treated with *Micrococcus luteus-*derived vesicles, NE secretion from neutrophils was suppressed in a dose-dependent manner. From the above results, it can be seen that *Micrococcus luteus-derived* vesicles can efficiently treat inflammatory diseases mediated by neutrophilic elastase due to the activation of neutrophils.

### Example 7. Evaluation of therapeutic effect of Micrococcus luteus-derived vesicles in mouse model of neutrophilic pulmonary disease

As illustrated in FIG. 9, a pulmonary disease mouse model was prepared by intranasally administering 10 ng/ml of *E. coli*-derived vesicles *(E. coli* EVs), which are pathogenic nanoparticles, to evaluate the effect of *Micrococcus luteus-derived* vesicles on pulmonary diseases, and therapeutic effects were evaluated by intranasally administering *Micrococcus luteus-derived* vesicles *(M. luteus* EVs) to the mouse model.

As illustrated in FIGS. 10A and 10B, the number of inflammatory cells in bronchoalveolar lavage fluid (BALF) and histological changes in the lungs were evaluated in order to evaluate the therapeutic effect on pulmonary inflammation. Specifically, for the number of inflammatory cells in the bronchoalveolar lavage fluid (BALF), the bronchoalveolar lavage fluid (BALF) was collected by connecting a syringe containing 1 mL of PBS to the airway, and then the total number of cells was measured using trypan blue (Abcam). In addition, in order to evaluate the histological changes of the lungs, a hematoxylin & eosin staining method was used, and specifically, after a slide glass was attached to Cytopro (ELItech) to fix the cells, the cells were stained with hematoxylin (DAKO) and eosin (Sigma, USA), and then the number of neutrophils was measured.

As a result, as illustrated in FIG. 10A, it was confirmed that the numbers of macrophages and neutrophils in bronchoalveolar lavage fluid (BALF) were decreased in a dose-dependent manner in the group to which *Micrococcus luteus-*derived vesicles were administered. Furthermore, as illustrated in FIG. 10B, it was confirmed that inflammatory cell infiltration was remarkably reduced in the pulmonary tissue of the group to which *Micrococcus luteus-derived* vesicles were administered.

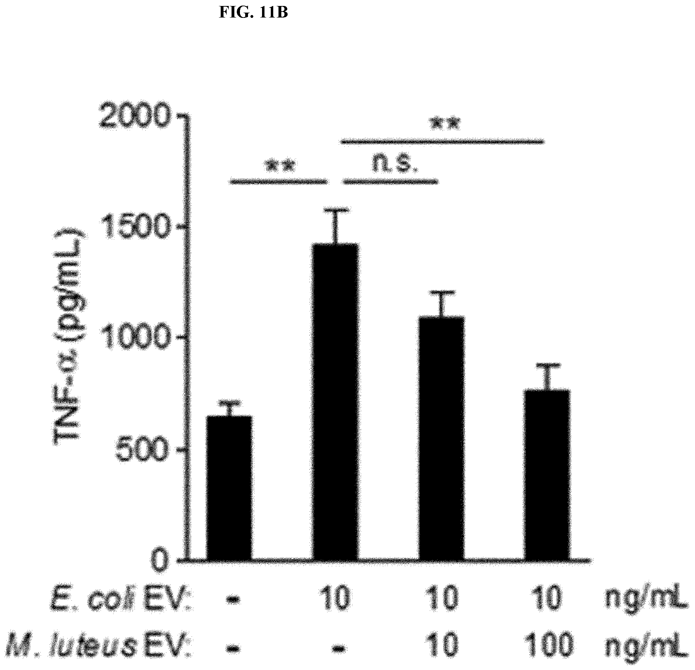

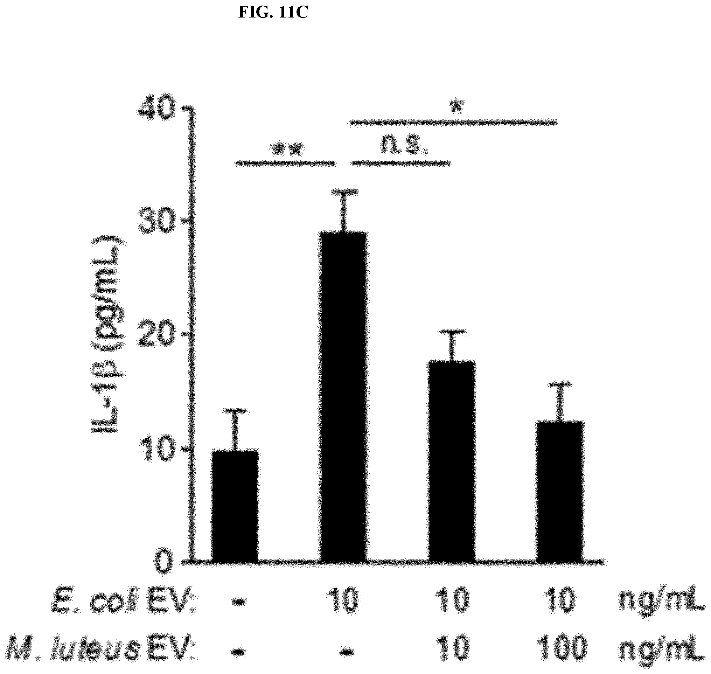

Further, as a result of evaluating the degree of secretion of inflammatory mediators in bronchoalveolar lavage fluid (BALF), as illustrated in FIGS. 11A to 11C, it was confirmed that the secretion of CXCL-1 (FIG. 11A) and TNF-α (FIG. 11B), and IL-1β (FIG. 11C) which are cytokines inducing inflammation, was suppressed in a dose-dependent manner by *Micrococcus luteus-derived* vesicles.

From the above results, it could be seen that neutrophilic pulmonary inflammatory diseases induced by pathogenic nanoparticles can be efficiently treated by *Micrococcus luteus-derived* vesicles.

### Example 8. Evaluation of acquired immunoregulatory effect of Micrococcus luteus-derived vesicles in neutrophilic pulmonary disease mouse model

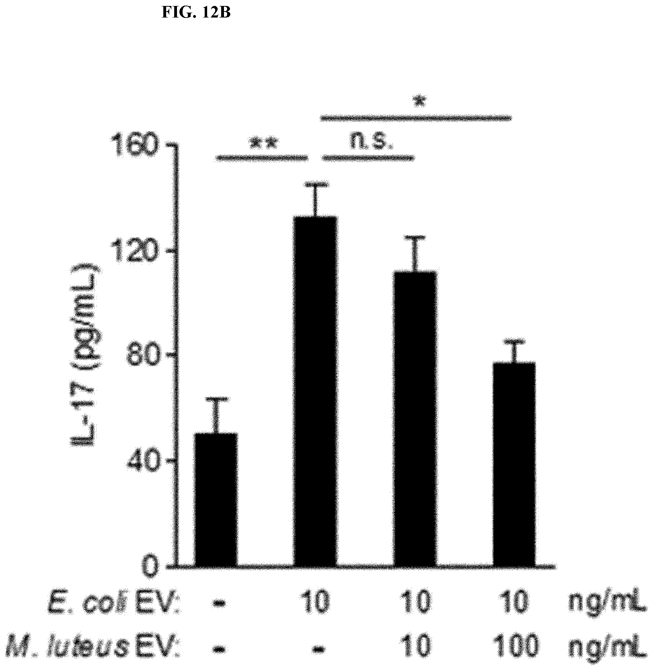

Immune function regulatory effects were evaluated by intranasally administering *Micrococcus luteus-derived* vesicles to the pulmonary disease mouse model of Example 7. As a result, as illustrated in FIGS. 12A and 12B, it was confirmed that the secretion of Th17 immune response-associated cytokines IL-6 (FIG. 12A) and IL-17 (FIG. 12B), which induce neutrophilic inflammation, was suppressed in a dose-dependent manner by *Micrococcus luteus-derived* vesicles.

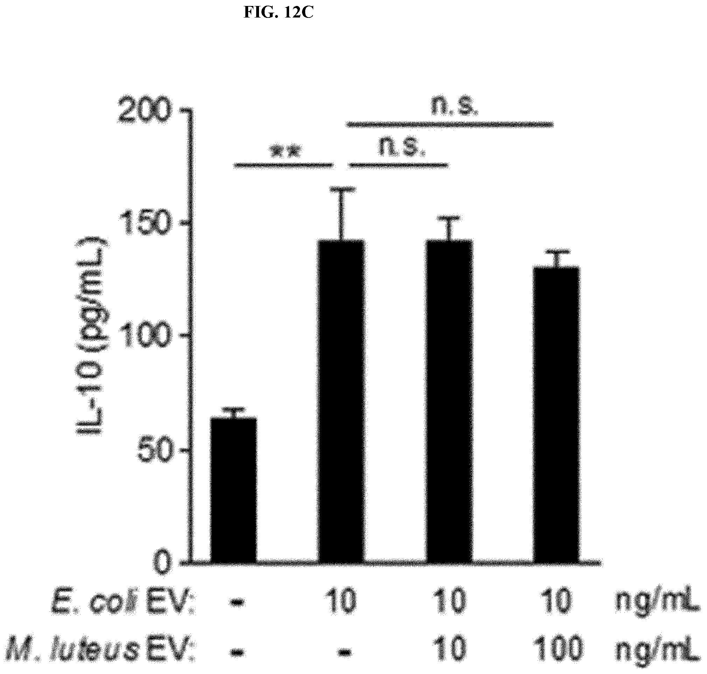

Meanwhile, as illustrated in FIG. 12C, the secretion of IL-10, which suppresses immune function, was not suppressed by *Micrococcus luteus-derived* vesicles.

From the above results, it can be seen that the anti-inflammatory effect of *Micrococcus luteus-derived* vesicles appears while suppressing the Th17 immune response induced by pathogenic nanoparticles.

### Example 9. Evaluation of airway hyperresponsiveness suppressive effect of Micrococcus luteus-derived vesicles in neutrophilic pulmonary disease mouse model

Inflammatory responses caused by immune dysfunction lead to functional changes in organs, resulting in lung dysfunction. Functional changes in lungs were evaluated by intranasally administering Micrococcus luteus-derived vesicles to the pulmonary disease mouse model of Example 7. Specifically, functional changes were evaluated by measuring airway hyperresponsiveness (AHR) due to methacholine using flexiVent (SCIREQ, Canada). That is, after aerosol methacholine (Sigma, USA) was administered to each mouse at various concentrations (0 mg/mL, 6.25 mg/mL, 12.5 mg/mL and 25 mg/mL), the maximal airway response to inhaled methacholine was measured.

As a result, as illustrated in FIG. 13, it was confirmed that airway hyperresponsiveness induced by methacholine was suppressed in a dose-dependent manner in the group to which *Micrococcus luteus-derived* vesicles were administered. From the above results, it can be seen that pulmonary disease-associated functional changes induced by pathogenic nanoparticles can be efficiently treated by *Micrococcus luteus-derived* vesicles.

### Example 10. Anti-inflammatory effect evaluation of Micrococcus luteus-derived vesicles in mouse model of pulmonary disease caused by LPS-contaminated allergens

When only protein antigens are inhaled, immune tolerance rather than immunologically hypersensitive responses occurs in the respiratory tract, and as a result, inflammatory diseases caused by allergens do not occur, but when bacterial causative factors such as lipopolysaccharide (LPS) and allergens are simultaneously inhaled, a hypersensitive response to the allergens occurs due to innate immune dysfunction caused by LPS. In particular, a low concentration of LPS causes eosinophilic asthma by a Th2 immune response, and a high concentration of LPS causes neutrophilic asthma by a Th17 immune response. As illustrated in FIG. 14, a neutrophilic pulmonary disease mouse model was prepared by intranasally administering an allergen (ovalbumin; OVA) together with a high concentration of LPS. Specifically, 75 µg of a protein antigen ovalbumin (OVA) and 10 µg of LPS were simultaneously administered intranasally for 4 days to induce hypersensitive response to OVA, and then 50 µg of OVA was intranasally administered for 3 weeks to prepare a mouse model of pulmonary disease caused by protein antigens. *Micrococcus luteus-derived* vesicles *(M. luteus* EVs) were intranasally administered simultaneously with OVA for 3 weeks to induce a disease with OVA. As a control drug, 20 µg of dexamethasone (Dex) was intraperitoneally administered.

As a result, as illustrated in FIGS. 15A and 15B, similar to the group to which dexamethasone was administered, in the group to which *Micrococcus luteus-derived* vesicles were administered, the number of inflammatory cells and the number of neutrophils in bronchoalveolar lavage fluid (BALF) were significantly reduced compared to the pulmonary disease positive control group induced by LPS-contaminated allergens.

In addition, as a result of evaluate the histological changes in the lungs using a hematoxylin & eosin staining method, as illustrated in FIG. 15C, it was confirmed that compared to the pulmonary disease positive group induced by LPS-contaminated allergens, the group to which *Micrococcus luteus-derived* vesicles were administered significantly suppressed inflammatory cell infiltration into the pulmonary tissue, similar to the group to which dexamethasone was administered.

Furthermore, IL-1β and IL-17 in bronchoalveolar lavage fluid were measured using ELISA (R&D Systems) in order to evaluate the immune regulatory effects of *Micrococcus luteus-derived* vesicles in the mouse model.

As a result, as illustrated in FIGS. 16A and 16B, it was confirmed that the concentrations of inflammatory cytokine IL-1β (FIG. 16A) and IL-17 (FIG. 16B), which is a Th17 immune response marker caused by a protein antigen, in bronchoalveolar lavage fluid (BALF) were significantly reduced by *Micrococcus luteus-derived* vesicles.

From the above results, it can be seen that a neutrophilic pulmonary disease caused by a Th17 immune response caused by allergens can be effectively treated by *Micrococcus luteus-derived* vesicles.

### Example 11. Immune regulatory mechanism analysis of Micrococcus luteus-derived vesicles

Innate immune responses to various stresses are known to be very important for disease pathogenesis. In particular, the NLRP3 protein present in the cytoplasm is known to be a key signaling pathway in the pathogenesis of various intractable diseases caused by immune dysfunction. Further, t-bet and ROR-γt, which are associated with the pathogenesis of immune dysfunction induced by protein antigens, are known to be key signaling materials in the generation of hypersensitive responses by Th1 cells and Th17 cells to antigens, respectively.

In order to evaluate the immune function regulatory effect of *Micrococcus luteus*-derived vesicles, pulmonary tissue in the mouse model of Example 10 was obtained, and then the expression of an innate immune-related protein NLRP3 and acquired immune-related proteins t-box protein expressed in T cells (t-bet) and retinoic acid receptor-related orphan nuclear receptor gamma (ROR-γt) was confirmed by western blotting. 50 µg of protein was used to measure the expression level of each protein.

As a result, as illustrated in FIG. 17, it was confirmed that in the group to which LPS-contaminated allergens were administered, NLRP3 expression was remarkably increased compared to the control group, and in the group to which *Micrococcus luteus-derived* vesicles were administered, NLRP3 expression was remarkably suppressed similarly to the group to which dexamethasone was administered. In addition, it was confirmed that in the group to which *Micrococcus luteus*-derived vesicles were administered, the expression of t-bet and ROR-γt was suppressed more efficiently compared to dexamethasone.

From the above results, it could be seen that *Micrococcus luteus-derived* vesicles suppress NLRP3 inflammasome signals upon the generation of Th1 and Th17 hypersensitive responses by LPS-contaminated allergens, thereby suppressing the formation of allergen-specific Th1 and Th17 cells to regulate immune function.

### Example 12. Evaluation of efficacy of Micrococcus luteus-derived vesicles on regulation of cellular homeostasis against oxidative stress

When cells are repeatedly exposed to various stresses, cellular senescence occurs due to oxidative stress in cells, and cell dysfunction and cell death are caused, leading to disease. In particular, a low concentration of nitric oxide (NO) generated through eNOS signals not only plays a key role in maintaining cellular homeostasis by antagonizing the action of reactive oxygen species (ROS), which are mainly responsible for oxidative stress, but also suppresses the development of airway hyperresponsiveness, which is an important functional change in pulmonary diseases.

In order to evaluate the effect of *Micrococcus luteus-derived* vesicles on eNOS signal activation, which is one of the defense mechanisms against oxidative stress, A549 cells were treated with *Micrococcus luteus-derived* vesicles by the method of Example 4, and then the expression pattern of eNOS signaling proteins was evaluated. As a specific method for evaluating the expression of signaling proteins, cells were lysed using a lysis buffer, proteins were extracted, and proteins were quantified using a BCA protein assay kit (Thermo, USA). 50 µg of protein per sample was electrophoresed in a 10% polyacrylamide gel, and the isolated proteins were transferred to a nitrocellulose membrane. After blocking with skim milk-added Tris-buffered saline containing 0.05% Tween 20 (TBST) at room temperature for 30 minutes, primary antibodies specific for p-ERK, ERK, eNOS, p-eNOS, and β-actin were diluted 1/1,000 and allowed to react at 4°C for 24 hours. Thereafter, the primary antibodies were washed three times with phosphate buffer saline containing 0.05% Tween-20 (PBST) for 10 minutes each, and allowed to react with a secondary antibody diluted at1/5,000 at room temperature for 1 hour. After washing five times with PBST for 10 minutes each, bands were confirmed using the ECL select reagent.

As a result, as illustrated in FIG. 18, upon treatment with E. coli-derived vesicles *(E. coli* EVs), which are pathogenic nanoparticles, eNOS signal activation was suppressed. In contrast, upon treatment with *Micrococcus luteus-derived* vesicles, the phosphorylation of ERK and eNOS was increased similarly to when dexamethasone (Dex) was administered. In addition, it was confirmed that eNOS phosphorylation by *Micrococcus luteus*-derived vesicles was suppressed when heat-treated *Micrococcus luteus-derived* vesicles were administered.

From the above results, it can be seen that *Micrococcus luteus-derived* vesicles not only induce low concentrations of NO by activating eNOS signals to increase cellular homeostasis, but also suppress the development of lung disease-related functional changes.

Through the above results, it could be seen that the *Micrococcus luteus-*derived vesicles of the present invention efficiently suppress the development of a neutrophilic pulmonary disease. In particular, it could be seen that the vesicles restore innate and acquired immune functions by suppressing NLRP3 protein expression and inflammasome production induced by oxidative stress, mitochondrial dysfunction, and lysosomal damage, and the like. In addition, it could be seen that eNOS signals induce the production of low-concentration NO to increase cellular homeostasis. In particular, it was confirmed that *Micrococcus luteus-derived* vesicles, when orally or intranasally administered, pass through the mucous membrane to be distributed in pulmonary tissue, so that the *Micrococcus luteus-*derived vesicles of the present invention are expected not only to be used for ameliorating, preventing, or treating neutrophilic pulmonary diseases, but also to be usefully used as a drug carrier for treating pulmonary diseases.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [industrial Applicability]

The present inventors confirmed that when *Micrococcus luteus-derived* vesicles were orally or intranasally administered, the vesicles passed through the mucous membrane and were delivered to the lungs. Furthermore, the present inventors confirmed that not only the secretion of inflammatory mediators by biological causative factors were significantly suppressed when epithelial cells and inflammatory cells were treated with the vesicles, but also the secretion of NLRP3 protein by the biological causative factors was remarkably suppressed when the cells were treated with the vesicles. Further, it was confirmed that when the vesicles were administered to a mouse model of neutrophilic disease caused by biological causative factors, neutrophilic pulmonary inflammation and functional changes were significantly suppressed, so that it is expected that *Micrococcus luteus-derived* vesicles according to the present invention will be able to be usefully used not only for developing a pharmaceutical or health functional food for preventing, ameliorating symptoms, or treating a neutrophilic pulmonary disease, but also as a drug delivery system for treating the disease.

## Claims

1. A pharmaceutical composition for preventing or treating a neutrophilic pulmonary disease, comprising *Micrococcus luteus*-derived vesicles as an active ingredient.

2. The pharmaceutical composition of claim 1, wherein the pulmonary disease is a pulmonary disease mediated by a nucleotide-binding oligomerization domain, leucine rich repeat and pyrin domain containing 3 (NLRP3) inflammasome.

3. The pharmaceutical composition of claim 1, wherein the pulmonary disease is one or more diseases selected from the group consisting of asthma, emphysema, cystic fibrosis (CF), bacterial pneumonia, viral pneumonia, idiopathic pulmonary fibrosis (IPF), interstitial pneumonitis, acute respiratory distress syndrome (ARDS), and acute lung injury (ALI).

4. The pharmaceutical composition of claim 3, wherein the asthma is neutrophilic asthma.

5. The pharmaceutical composition of claim 1, wherein the vesicles have an average diameter of 10 to 200 nm.

6. The pharmaceutical composition of claim 1, wherein the vesicles are naturally secreted or artificially produced from *Micrococcus luteus.*

7. The pharmaceutical composition of claim 1, wherein the composition suppresses the activity of the NLRP3 inflammasome.

8. A food composition for preventing or alleviating a neutrophilic pulmonary disease, comprising *Micrococcus luteus-derived* vesicles as an active ingredient.

9. The food composition of claim 8, wherein the pulmonary disease is one or more diseases selected from the group consisting of asthma, emphysema, cystic fibrosis (CF), bacterial pneumonia, viral pneumonia, idiopathic pulmonary fibrosis (IPF), interstitial pneumonitis, acute respiratory distress syndrome (ARDS), and acute lung injury (ALI).

10. An inhalant composition for preventing or treating a neutrophilic pulmonary disease, comprising *Micrococcus luteus-derived* vesicles as an active ingredient.

11. The inhalant composition of claim 10, wherein wherein the pulmonary disease is one or more diseases selected from the group consisting of asthma, emphysema, cystic fibrosis (CF), bacterial pneumonia, viral pneumonia, idiopathic pulmonary fibrosis (IPF), interstitial pneumonitis, acute respiratory distress syndrome (ARDS), and acute lung injury (ALI).

12. A quasi-drug composition for preventing or alleviating a neutrophilic pulmonary disease, comprising *Micrococcus luteus*-derived vesicles as an active ingredient.

13. A composition for delivering a drug for treating a neutrophilic pulmonary disease, comprising *Micrococcus luteus-derived* vesicles as an active ingredient.

14. A method for preventing or treating a neutrophilic pulmonary disease, the method comprising administering a composition comprising *Micrococcus luteus-*derived vesicles as an active ingredient to a subject in need thereof.

15. A use of a composition comprising *Micrococcus luteus-derived* vesicles as an active ingredient for preventing or treating a neutrophilic pulmonary disease.

16. A use of *Micrococcus luteus*-derived vesicles for preparing a drug for treating a neutrophilic pulmonary disease.

17. A method for delivering a drug for treating a neutrophilic pulmonary disease, the method comprising administering a composition comprising *Micrococcus luteus-*derived vesicles, which carry a drug for treating a neutrophilic pulmonary disease, as an active ingredient to a subject in need thereof.

18. A use of a composition comprising *Micrococcus luteus-derived* vesicles as an active ingredient for delivering a drug for treating a neutrophilic pulmonary disease.
